(19) Europäisches Patentamt
European Patent Office
Office européen des brevets

(11) Numéro de publication: **0 015 786**
**B1**

(12) **FASCICULE DE BREVET EUROPEEN**

(45) Date de publication du fascicule du brevet: **21.04.82**

(21) Numéro de dépôt: **80400049.5**

(22) Date de dépôt: **15.01.80**

(51) Int. Cl.³: **C 07 D 471/14,**
A 61 K 31/435
//(C07D471/14, 239/00,
221/00, 209/00),
(C07D471/14, 235/00,
221/00, 209/00)

(54) **Imidazo et pyrimido-pyrido-indoles, leur préparation et médicaments les contenant.**

(30) Priorité: **05.02.79 FR 7902839**

(43) Date de publication de la demande:
**17.09.80 Bulletin 80/19**

(45) Mention de la délivrance du brevet:
**21.04.82 Bulletin 82/16**

(84) Etats contractants désignés:
**AT BE CH DE FR GB IT LU NL SE**

(56) Documents cités:
**CHEMICAL ABSTRACTS, vol. 59, 1963 ref. 11479g Columbus, Ohio, US**
**G. de STEVENS et al.: "Synthesis of nitrogen-containing heterocyclic compounds"**

**CHEMICAL ABSTRACTS, vol 75, 1971 page 221, ref. 118318m Columbus, Ohio, US**

**THE JOURNAL OF ORGANIC CHEMISTRY vol. 27 (1962) Easton, PA, US**
**G. de STEVENS et al.: "Investigations in Heterocycles. XI. Tetracyclic and Pentacyclic Indolo (2,3-$\alpha$) quinolizines", pages 2457—62**

(73) Titulaire: **SYNTHELABO**
**58 rue de la Glacière**
**F-75013 Paris (FR)**

(72) Inventeur: **Koletar, Gabor Istvan**
**212 Dogwood Lane**
**Berkeley Heights, New Jersey 07922 (US)**
Inventeur: **Dupont, Régis**
**141, Bd de la Gare**
**F-75013 Paris (FR)**
Inventeur: **Lardenois, Patrick**
**18, Rue Varengue**
**F-92340 Bourg La Reine (FR)**
Inventeur: **Frost, Jonathan**
**122, Résidence "Les Eaux Vives"**
**F-91120 Palaiseau (FR)**
Inventeur: **Najer, Henry**
**2, avenue Emile Acollas**
**F-75007 Paris (FR)**

(74) Mandataire: **Thouret-Lemaitre, Elisabeth**
**Service Brevets - SYNTHELABO 58, rue de la Glacière**
**F-75621 Paris Cedex 13 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 015 786 B1

# Imidazo et pyrimido-pyrido-indoles, leur préparation et médicaments les contenant

La présente invention concerne des imidazo et pyrimido-pyrido-indoles, sous forme de racémates ou d'énantiomères, leurs sels d'addition aux acides pharmaceutiquement acceptables, leur préparation et leur application en thérapeutique.

Certains imidazo et pyrimido-pyrido-indoles ont déjà été décrits dans la littérature, notamment dans la demande japonaise 71 27 196, et dans les articles de G. DE STEVENS et al. (Oesterr. Chemiker-Ztg. 63 (6), 177—82 (1962), et J. Org. Chem. *27*, 2457—62 (1962).

Les composés de l'invention répondent à la formule (I)

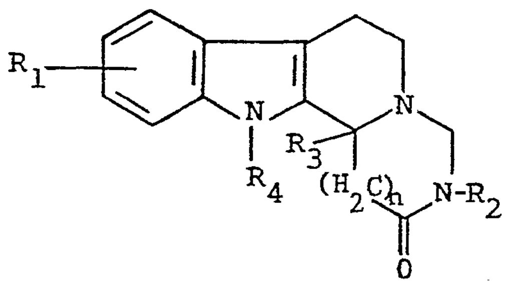

(I)

dans laquelle

n est 1 ou 0,

$R_1$ est un atome d'hydrogène ou d'halogène, un radical alkyle ou alcoxy ou le groupe $CF_3$,

$R_2$ est un radical alkyle, cycloalkyle, cycloalkylalkyle, phényle ou benzyle, ces deux derniers radicaux portant éventuellement un atome d'halogène, un radical alkyle ou alcoxy,

$R_3$ est un atome d'hydrogène ou un radical alkyle,

$R_4$ est un atome d'hydrogène ou un radical alkyle,

les alkyles et alcoxy ayant de 1 à 4 atomes de carbone, à l'exception du composé pour lequel $n = 1$, $R_1 = R_3 = R_4 = H$ et $R_2 = CH_3$.

Les composés de l'invention possèdent un carbone asymétrique en position 12b ($n = 1$) ou 11b ($n=0$).

Les racémates et les énantiomères font partie de l'invention. Les composés préférés de l'invention sont ceux pour lesquels $n=1$, $R_1$ est un atome d'hydrogène, un atome de chlore ou de fluor ou le radical méthyle, $R_2$ est le radical méthyle ou éthyle, $R_3$ est un atome d'hydrogène ou le radical méthyle et $R_4$ est un atome d'hydrogène ou le radical méthyle.

Selon l'invention on prépare les composés (I) à partir d'un composé de formule (II)

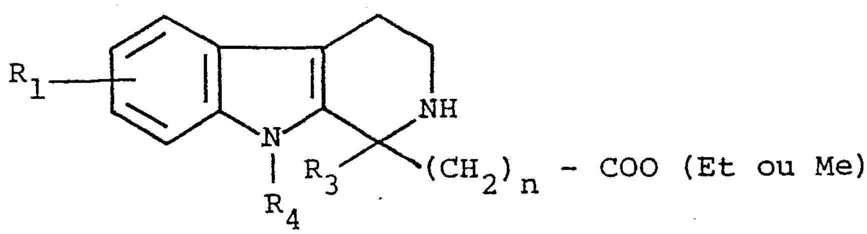

(II)

que l'on fait réagir avec une amine $R_2NH_2$, dans un solvant tel qu'un alcool ou l'amine $R_2NH_2$ elle-même, à une température allant de la température ambiante à 100°C, puis on cyclise le composé intermédiaire obtenu de formule (III)

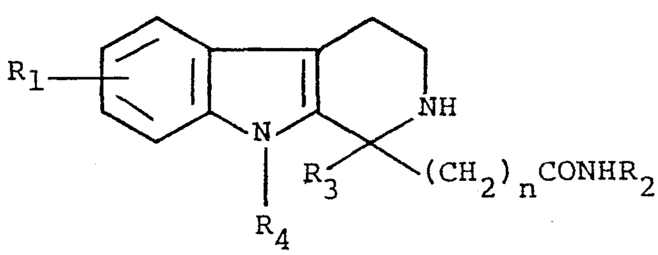

(III)

à l'aide de formaldéhyde en solution à 30% dans de l'eau pour obtenir les composés de formule (I).

Lorsque dans le composé (I), $R_4$ est un atome d'hydrogène, on peut alors l'alkyler pour obtenir un dérivé (I) dans lequel $R_4$ est un alkyle.

Les composés de départ (II) dans lesquels $R_3$ = alkyle sont préparés à partir de la tryptamine substituée ou non selon le schéma suivant:

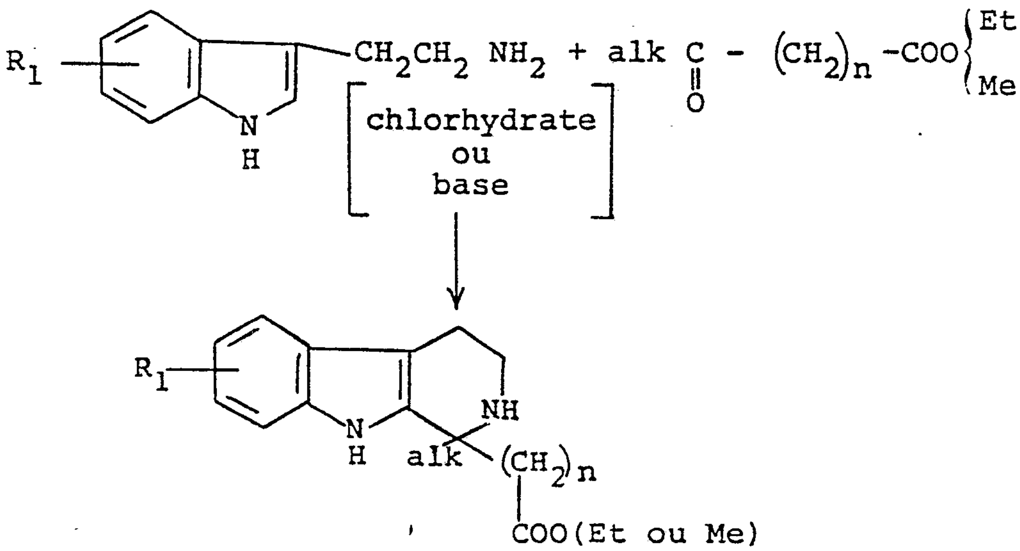


(II)

Cette réaction est décrite dans la littérature par J. A. MACLAREN, Aust. J. Chem. (1977) *30* 2045—51.

Les composés de départ (II) dans lesquels $R_3$ = H et n = 1 sont préparés à partir du chlorhydrate de tryptamine substituée ou non, selon le schéma suivant:

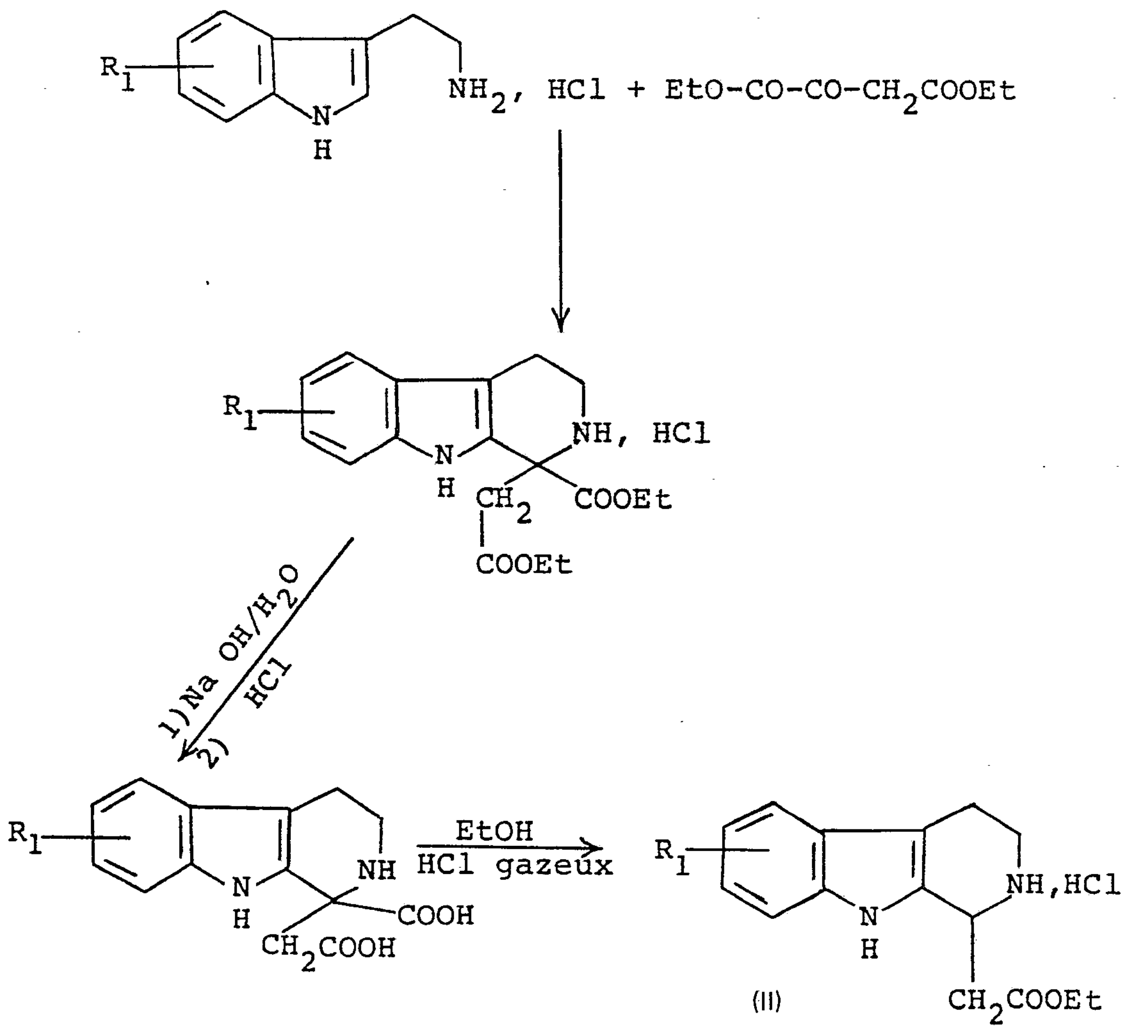


(II)

Le composé (II) pour lequel $R_1$ est H et n est 1 est décrit par L. H. GROVES and G. A. SWAN J. C. S. (1952) 650.

Les composés de départ (II) dans lesquels $R_1$ est différent de H sont nouveaux.

*Exemple de préparation d'un composé (II)*

$[R_1 = CH_3 - 9\ n = 1\ R_3 = H]$

1. On met en suspension dans 250 ml d'éthanol, 52,62 g (0,25 mole) de chlorhydrate de méthyl-5 tryptamine et on porte à reflux. On met en suspension dans 250 ml d'éthanol, 57,75 g d'éthoxy-carbonyl-3 dioxo-1,2 éthoxy-1 propane et on ajoute goutte à goutte en 10 mn, 25 ml d'acide chlorhydrique concentré. On ajoute cette suspension à la suspension de méthyl-5-tryptamine maintenue à la température de reflux. On laisse refroidir pendant la nuit. On élimine le solvant par évaporation et on dissout le résidu dans 400 ml d'eau et on alcalinise avec de l'ammoniaque. Après extraction avec de l'acétate d'éthyle, on obtient une huile que l'on chromatographie sur une colonne de silice. Après élution avec un mélange 8/2 de chloroforme et d'éthanol, on obtient une huile qui se solidifie par trituration avec de l'éther de pétrole. Après recristallisation dans de l'hexane, le composé obtenu

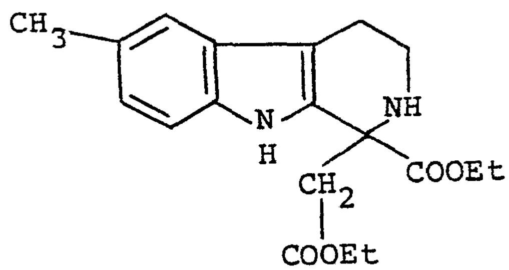

fond à 102—103°C.

2. On chauffe à reflux 45 g du composé précédent dans 450 ml d'une solution aqueuse à 10% de NaOH, pendant 20 h. On ajoute goutte à goutte, de l'acide chlorhydrique concentré (100 ml), en 30 mn, au mélange réactionnel refroidi. On filtre le solide obtenu et on le sèche sur $P_2O_5$.

3. On chauffe au reflux 99,6 g du solide brut précédemment obtenu dans un mélange de 250 ml d'éthanol et de 20 ml d'acide sulfurique concentré, pendant 9 h. On laisse reposer la nuit. On élimine l'éthanol par évaporation et on alcalinise le solide résiduel avec de l'ammoniaque. On extrait avec 3 fois 300 ml d'acétate d'éthyle la solution basique. On évapore. On obtient une huile qui, par trituration avec de l'éther de pétrole, donne un solide blanc. On le filtre et le sèche.

Après recristallisation dans de l'hexane, le composé (II) obtenu fond à 103°C.

Les exemples suivants illustrent l'invention.

Les micro-analyses et les spectres IR et RMN confirment la structure des composés.

Exemple 1

Diméthyl-2,11b hexahydro-2,3,5,6,11,11b 1H-imidazo[1',5':1,2]pyrido[3,4-b]indolone-1.

$[n = 0,\ R_1 = H,\ R_2 = CH_3,\ R_3 = CH_3,\ R_4 = H]$

1. Méthylaminocarbonyl-1 méthyl-1 tétrahydro-2,3,4,9 1H-pyrido[3,4-b]indole.

On met 4,8 g (0,02 mole) de méthyl-1 tétrahydro-2,3,4,9 1H-pyrido[3,4-b]indole-1-carboxylate de méthyle (composé de départ II) en solution dans 100 ml d'éthanol saturé de méthylamine.

On laisse à la température ambiante pendant 48 heures. On élimine le solvant puis reprend le résidu par 20 ml d'éthanol. On filtre et lave à l'éthanol.

F = 230—231°C.

2. Diméthyl-2,11b hexahydro-2,3,5,6,11,11b 1H-imidazol[1',5':1,2]pyrido[3,4-b]indolone-1

On place 12 g (0,05 mole) du composé obtenu précédemment dans 200 ml d'éthanol. On ajoute 3 g de KOH en pastilles puis 25 ml d'une solution aqueuse à 30% de formaldéhyde.

On agite à 70°C pendant 12 heures.

On filtre le précipité et le lave à l'éthanol. Après recristallisation dans du propanol le composé fond à 252—253°C.

Exemple 2

Chloro-9 méthyl-3 hexahydro-3,4,6,7,12,12b pyrimido[1',6':1,2]pyrido[3,4-b]indol-2(1H)-one

$[n = 1,\ R_1 = Cl - 9,\ R_2 = CH_3,\ R_3 = H,\ R_4 = H]$

1. N-méthyl chloro-6 tétrahydro-2,3,4,9 1H-pyrido[3,4-b]indole-acétamide-1.

On dissout 8 g de chloro-6 tétrahydro-2,3,4,9 1H-pyrido[3,4-b]indole-1-acétate d'éthyle (composé de départ (II) dans un mélange de 150 ml d'éthanol et de 150 ml de méthylamine à 33% dans l'éthanol. On chauffe le mélange réactionnel à 200° pendant 7 heures dans un autoclave. On le laisse refroidir pendant la nuit. On évapore la solution.

Le solide qui est recristallisé dans de l'éthanol fond à 228—230°C.

2. Chloro-9 méthyl-3 hexahydro-3,4,6,7,12,12b pyrimido[1',6':1,2]pyrido[3,4-b]indol-2 (1H)-one

On dissout 1,7 g du composé obtenu précédemment dans un mélange de 20 ml d'éthanol et 2 ml de formaldéhyde à 30% dans de l'eau à la température ambiante. On agite pendant 30 mn. On élimine le solvant et on reprend le solide blanc avec de l'éthanol froid et on filtre. On recristallise le produit dans de l'éthanol.

F = 257°C.

Exemple 3

Diméthyl-3,12b hexahydro-3,4,6,7,12,12b pyrimido[1',6':1,2]pyrido[3,4-b]indol-2(1H)-one

[$n = 1$, $R_1 = H$, $R_2 = CH_3$, $R_3 = CH_3$, $R_4 = H$]

1. N-méthyl méthyl-1 tétrahydro-2,3,4,9 pyrido[3,4-b]indole-acétamide-1.

On introduit 36,9 g de méthyl-1 tétrahydro-2,3,4,9 1H-pyrido[3,4-b]indole-1-carboxylate d'éthyle dans un autoclave avec 300 ml d'une solution de $CH_3NH_2$ à 33% dans de l'éthanol. On chauffe à 100°C pendant 8 heures. On chasse le solvant et fait cristalliser le résidu dans de l'éthanol.

F = 182—183°C.

2. Diméthyl-3,12b hexahydro-3,4,6,7,12,12b pyrimido[1',6':1,2]pyrido 3,4-b]indol-2 (1H)-one.

On introduit dans un ballon 20 g du composé précédent et 150 ml d'éthanol. On ajoute une solution de formaldéhyde à 33% dans de l'eau et on agite à la température ambiante pendant 3 heures On chasse le solvant et on sèche le résidu par entraînement azéotropique avec du toluène. On dissout le résidu dans 500 ml de chloroforme et on sèche sur $Na_2SO_4$. Après évaporation de la solution on obtient une huile. On ajoute du tétrahydrofuranne sec puis on le chasse. On obtient un solide blanc que l'on fait recristalliser dans de l'acétate d'éthyle.

F = 204°C.

Exemple 4

Triméthyl-3,12,12b hexahydro-3,4,6,7,12,12b pyrimido[1',6':1,2]pyrido[3,4-b]indol-2(1H)-one

[$n = 1$, $R_1 = H$, $R_2 = CH_3$, $R_3 = CH_3$, $R_4 = CH_3$]

On introduit dans un ballon 3 g du composé obtenu dans l'exemple 2 et 30 ml de diméthylformamide. On ajoute 0,53 g d'hydrure de sodium et on agite à la température ambiante pendant 2 heures. On ajoute 0,9 ml d'iodure de méthyle et on agite 1 mn à la température ambiante. On chasse le solvant et on chromatographie le résidu sur $SiO_2$. On élue avec un mélange 8/2 $CHCl_3$/EtOH. On obtient un solide blanc que l'on recristallise dans $CH_3OCH_2CH_2OCH_3$.

F = 159—160°C

Les composés de l'invention préparés à titre d'exemples sont représentés dans le tableau I suivant.

TABLEAU I

| Composé n° | n | $R_1$ | $R_2$ | $R_3$ | $R_4$ | Caractéristiques F (°C) |
|---|---|---|---|---|---|---|
| 1 | 0 | H | $CH_3$ | $CH_3$ | H | 252–3 |
| 2 | 1 | H | $nC_4H_9$ | H | H | 232 |
| 3 | 1 | H | 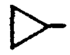 | H | H | 233 |
| 4 | 1 | H | $C_6H_5CH_2$ | H | H | 204 |
| 5 | 1 | Cl–9 | $CH_3$ | H | H | 257 |
| 6 | 1 | F–9 | $CH_3$ | H | H | 223 |
| 7 | 1 | H | $CH_3$ | H | $CH_3$ | 165–6 |
| 8 | 1 | $CH_3$–9 | $CH_3$ | H | H | 225 |
| 9 | 1 | $CH_3$–9 | $CH_3$ | H | $CH_3$ | 136–8 |
| 10 | 1 | H | $CH_3$ | $CH_3$ | $CH_3$ | 160 |
| 11 | 1 | H | $CH_3$ | $CH_3$ | H | 204 |
| 12 | 1 | H | $C_2H_5$ | H | H | 227 |
| 13 | 1 | $CH_3O$–9 | $CH_3$ | H | H | 224 |
| 14 | 1 | H | 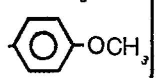 | H | H | >260 |
| 15 | 1 | Cl–9 | $C_2H_5$ | H | H | 256–7 |
| 16 | 1 | Cl–9 | 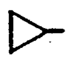 | H | H | 264 |
| 17 | 0 | F–8 | $C_2H_5$ | $CH_3$ | H | 207 |
| 18 | 0 | H | $C_2H_5$ | $CH_3$ | H | 205 |
| 19 | 0 | F–8 | $CH_3$ | $CH_3$ | H | 223 |
| 20 | 0 | Cl–8 | $CH_3$ | $CH_3$ | H | 248 |
| 21 | 0 | F–8 | 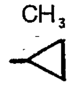 | $CH_3$ | H | 215 |
| 22 | 0 | H | 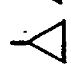 | $CH_3$ | H | 214 |
| 23 | 1 | $CF_3$–9 | $CH_3$ | H | H | 230 |

Les composés de l'invention ont été soumis à des essais pharmacologiques au cours desquels ils se sont révélés actifs comme anticonvulsivants.

La toxicité aigüe a été déterminée chez la souris par voie intrapéritonéale. La DL varie de 200 à 2000 mg/kg.

L'activité anticonvulsivante a été déterminée par le test de l'antagonisme vis-à-vis des convulsions induites par la bicuculline chez la souris (Perez de la Mora, M. and Tapia R. (1973), Biochem. Pharmacol. *22*, 2635—2639).

La $DA_{50}$ des composés de l'invention varie entre 30 et 50 mg/kg par voie i.p.

Les résultats précédents montrent que les composés peuvent être utilisés pour le traitement des épilepsies.

Les composés de l'invention peuvent être présentés sous toute forme appropriée pour l'administration par voie orale, parentérale, endorectale, par exemple sous forme de comprimés, de dragées, de gélules, de solutions buvables ou injectables, etc... avec tout excipient approprié.

La posologie quotidienne peut aller de 200 à 1500 mg.

**Revendications pour les Etats contractants: BE, CH, DE, FR, GB, IT, LU, NL, SE**

1. Imidazo et pyrimido-pyrido-indoles sous la forme de racémates ou d'énantiomères répondant à la formule (I)

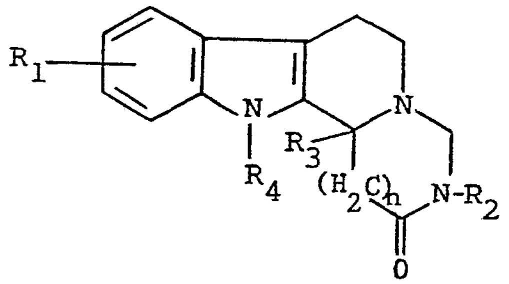

(I)

dans laquelle
n est 1 ou 0,
$R_1$ est un atome d'hydrogène ou d'halogène, un radical alkyle ou alcoxy ou le groupe $CF_3$,
$R_2$ est un radical alkyle, cycloalkyle, cycloalkylalkyle, phényle, ou benzyle, ces deux derniers radicaux portant éventuellement un atome d'halogène, un radical alkyle ou alcoxy,
$R_3$ est un atome d'hydrogène ou un radical alkyle,
$R_4$ est un atome d'hydrogène ou un radical alkyle,
les alkyles et alcoxy ayant de 1 à 4 atomes de carbone, à l'exception du composé pour lequel $n = 1$, $R_1 = R_3 = R_4 = H$ et $R_2 = CH_3$.

2. Dérivés selon la revendication 1, dans lesquels n est 1.

3. Dérivés selon la revendication 2, caractérisés par le fait que $R_1$ est un atome d'hydrogène, un atome de chlore ou de fluor ou le radical méthyle,
$R_2$ est le radical méthyle ou éthyle, $R_3$ est un atome d'hydrogène ou le radical méthyle et $R_4$ est un atome d'hydrogène ou le radical méthyle.

4. Procédé de préparation des composés de la revendication 1, procédé caractérisé en ce qu'on fait réagir un composé de formule (II)

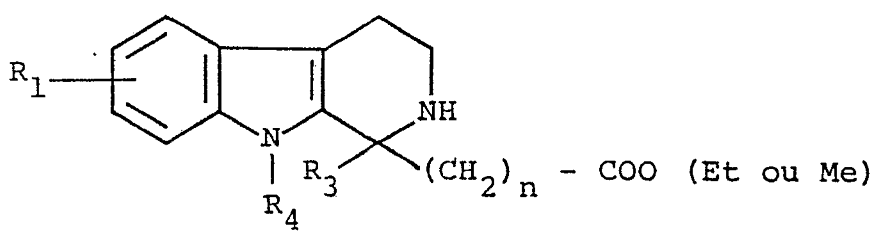

(II)

avec un amine $R_2NH_2$ puis on cyclise le composé intermédiaire obtenu de formule (III)

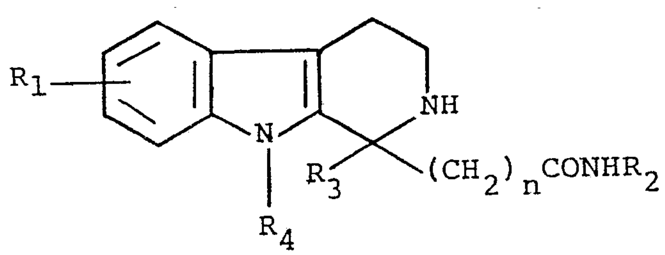

(III)

à l'aide de formaldéhyde en solution à 30% dans de l'eau, et, lorsque $R_4 = H$, on peut alkyler le composé (I) correspondant pour obtenir un composé (I) dans lequel $R_4$ est un alkyle, les radicaux ayant les définitions données dans la revendication 1.

5. Médicament caractérisé en ce qu'il contient un composé tel que spécifié dans l'une quelconque des revendications 1 à 3.

**Patentansprüche für die Vertragsstaaten: BE, CH, DE, FR, GB, IT, LU, NL, SE.**

1. Imidazo- und Pyrimido-Pyrido-Indole in Form derer Racemate oder Enantiomere entsprechend der Formel (I)

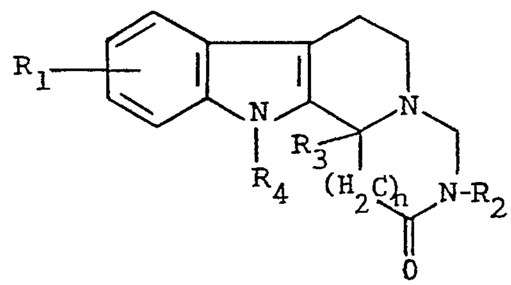

worin
n für 1 oder 0,
$R_1$ für ein Wasserstoff — oder Halogenatom, einen Alkyl- oder Alkoxy- oder $CF_3$-rest.
$R_2$ für einen Alkyl-, Cycloalkyl-, Cycloalkylalkyl-, Phenyl- oder Benzylrest, wobei die zwei letzteren gegebenenfalls ein Halogenatom oder einen Alkyl- oder Alkoxyrest tragen können,
$R_3$ für ein Wasserstoffatom oder einen Alkylrest,
$R_4$ für ein Wasserstoffatom oder einen Alkylrest steht,
wobei die Alkyl- und Alkoxyreste 1 bis 4 Kohlenstoffatome aufweisen, mit Ausnahme der Verbindung für welche $n = 1$, $R_1 = R_3 = R_4 = H$ und $R_2 = CH_3$.

2. Derivate gemäss Anspruch 1, worin n für 1 steht.

3. Derivate gemäss Anspruch 2, dadurch gekennzeichnet, dass $R_1$ für ein Wasserstoffatom, ein Chloratom, ein Fluoratom oder einen Methylrest, $R_2$ für einen Methyl- oder Äthylrest, $R_3$ für ein Wasserstoffatom oder einen Methylrest und $R_4$ für ein Wasserstoffatom oder einen Methylrest steht.

4. Verfahren zur Herstellung der Verbindungen des Anspruchs 1, dadurch gekennzeichnet, dass man eine Verbindung der Formel (II)

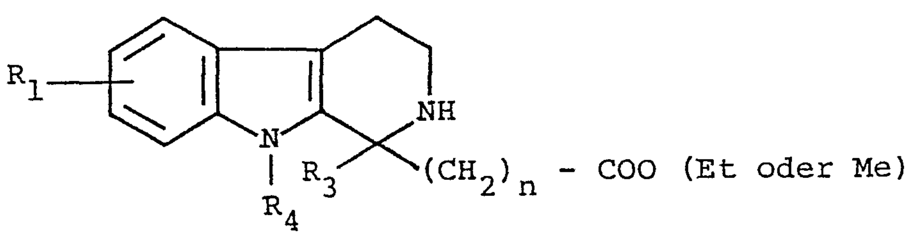

(II)

mit einen Amin $R_2NH_2$ reagieren lässt, dann die erhaltene Zwischenverbindung der Formel (III)

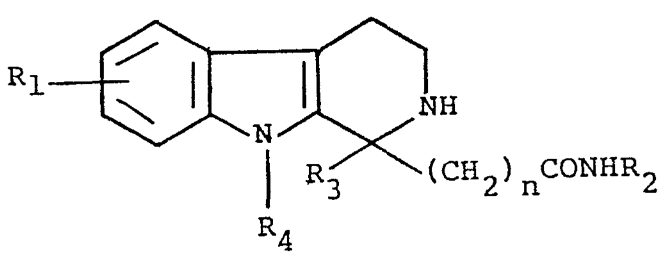

(III)

mittels 30% wässrigem Formaldehyd cyclisiert, und, wenn $R_4 = H$, die entsprechende Verbindung (I) alkylieren kann, um eine Verbindung (I) zu erhalten, worin $R_4$ für Alkyl steht, wobei die Reste die in Anspruch 1 angegebenen Bedeutungen haben.

5. Arzneimittel dadurch gekennzeichnet, das es eine Verbindung gemäss irgendeinem der Ansprüche 1 bis 3 enthält.

**Claims for the Contracting States: BE, CH, DE, FR, GB, IT, LU, NL, SE.**

1. Imidazo and pyrimido-pyrido-indoles in form of racemates or enantiomers corresponding to formula (I)

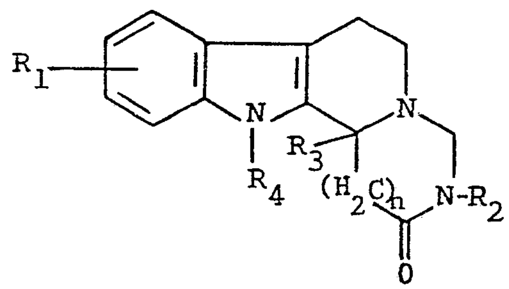

(I)

wherein
n is 1 or 0,
$R_1$ is a hydrogen or halogen atom, an alkyl or alkoxy radical or a $CF_3$ group,
$R_2$ is an alkyl, cycloalkyl, cycloalkylalkyl, phenyl or benzyl radical, the two latter radicals being optionally substituted with a halogen atom or an alkyl or alkoxygroup,
$R_3$ is a hydrogen atom or an alkyl radical,
$R_4$ is a hydrogen atom or an alkyl radical,
said alkyl and alkoxy radicals having 1 to 4 carbon atoms, with the exception of the compound for which $n = 1$, $R_1 = R_3 = R_4 = H$ and $R_2 = CH_3$.

2. Derivatives according to claim 1, wherein n is 1.

3. Derivatives according to claim 2, characterized in that $R_1$ is a hydrogen atom, a chlorine atom, a fluorine atom or a methyl radical, $R_2$ is a methyl or ethyl radical, $R_3$ is a hydrogen atom or a methyl radical and $R_4$ is a hydrogen atom or a methyl radical.

4. A process for preparing the compounds of claim 1 characterized in that a compound of the formula (II)

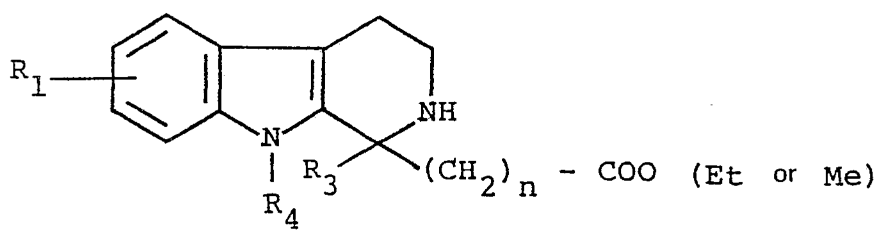

(II)

is reacted with an amine $R_2NH_2$, then the intermediate compound obtained, of formula (III)

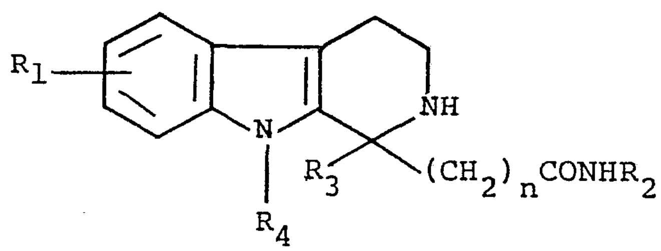

(III)

is cyclized by means of formaldehyde in 30% aqueous solution, and, when $R_4 = H$, the corresponding compound (I) may be alkylated to yield a compound (I) wherein $R_4$ is alkyl, the radicals having the meanings given in claim 1.

5. A drug characterized in that it contains a compound as specified in any one of claims 1 to 3.

**Revendication pour l'Etat contractant: AT**

Procédé de préparation d'imidazo et pyrimido-pyrido-indoles sous la forme de racémates ou d'énantiomères répondant à la formule (I)

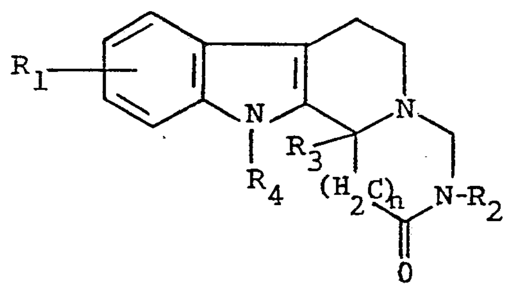

dans laquelle
n est 1 ou 0,
$R_1$ est un atome d'hydrogène ou d'halogène, un radical alkyle ou alcoxy ou le groupe $CF_3$,
$R_2$ est un radical alkyle, cycloalkyle, cycloalkylalkyle, phényle ou benzyle ces deux derniers radicaux portant éventuellement un atome d'halogène, un radical alkyle ou alcoxy,
$R_3$ est un atome d'hydrogène ou un radical alkyle,
$R_4$ est un atome d'hydrogène ou un radical alkyle,
les alkyles et alcoxy ayant de 1 à 4 atomes de carbone, à l'exception du composé pour lequel n = 1, $R_1 = R_3 = R_4 = H$ et $R_2 = CH_3$,
procédé caractérisé en ce qu'on fait réagir un composé de formule (II)

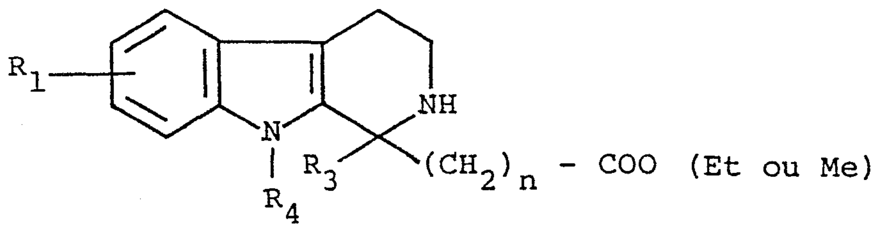

(II)

avec une amine $R_2NH_2$ puis on cyclise le composé intermédiaire obtenu de formule (III)

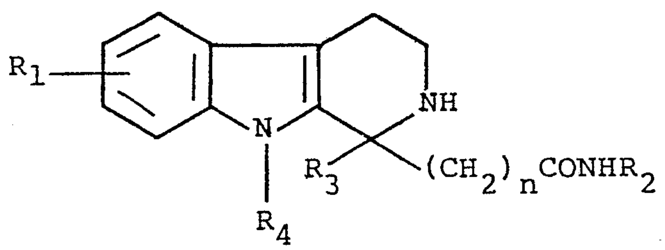

(III)

à l'aide de formaldéhyde en solution à 30% dans de l'eau, et, lorsque $R_4 = H$, on peut alkyler le composé (I) correspondant pour obtenir un composé (I) dans lequel $R_4$ est un alkyle.

**Patentanspruch für den Vertragsstaat: AT**

Verfahren zur Herstellung von Imidazo- und Pyrimido-Pyrido-Indolen in Form derer Racemate oder Enantiomere entsprechend der Formel (I)

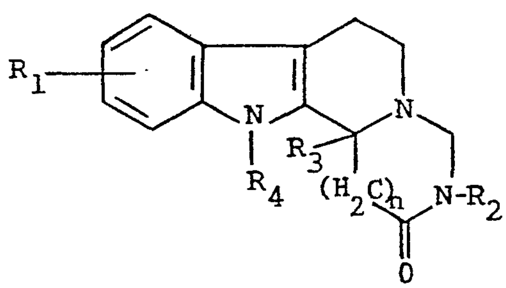

worin
n für 1 oder 0,
$R_1$ für ein Wasserstoff- oder Halogenatom, einen Alkyl- oder Alkoxy- oder $CF_3$-rest,
$R_2$ für einen Alkyl-, Cycloalkyl-, Cycloalkylalkyl-, Phenyl- oder Benzylrest, wobei die zwei letzteren gegebenenfalls ein Halogenatom oder einen Alkyl- oder Alkoxyrest tragen können,
$R_3$ für ein Wasserstoffatom oder einen Alkylrest,
$R_4$ für ein Wasserstoffatom oder einen Alkylrest steht,
wobei die Alkyl- und Alkoxyreste 1 bis 4 Kohlenstoffatome aufweisen,
mit Ausnahme der Verbindung für welche n = 1, $R_1 = R_3 = R_4 = H$ und $R_2 = CH_3$,
dadurch gekennzeichnet, dass man eine Verbindung der Formel (II)

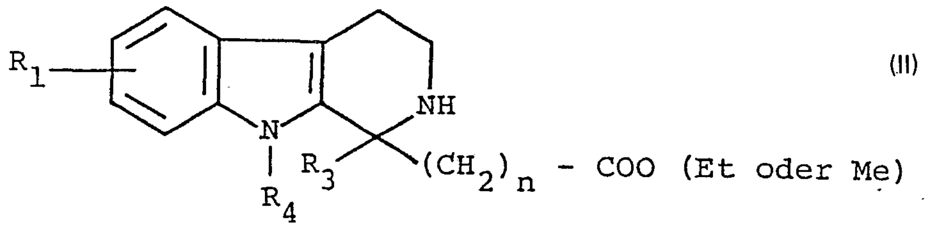

(II)

mit einen Amin $R_2NH_2$ reagieren lässt, dann die erhaltene Zwischenverbindung der Formel (III)

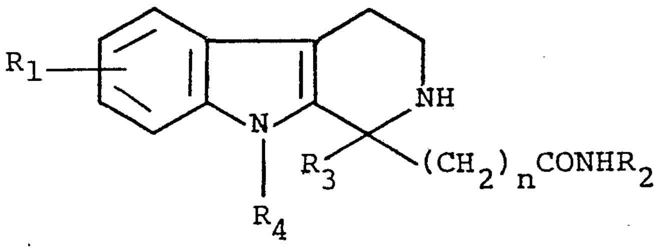

(III)

mittels 30% wässrigem Formaldehyd cyclisiert, und, wenn $R_4 = H$, die entsprechende Verbindung (I) alkylieren kann, um eine Verbindung (I) zu erhalten, worin $R_4$ für Alkyl steht.

**Claim for the Contracting State: AT**

A process for preparing imidazo and pyrimido-pyrido-indoles in form of racemates or enantiomers corresponding to formula (I)

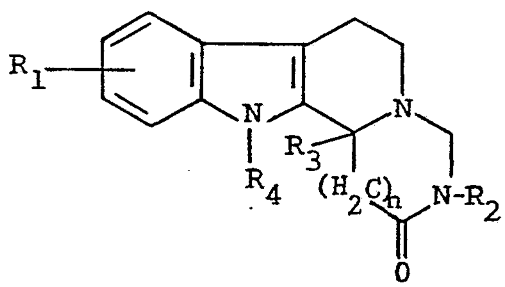

(I)

wherein
n is 1 or 0,
$R_1$ is a hydrogen or halogen atom, an alkyl or alkoxy radical or a $CF_3$ group,
$R_2$ is an alkyl, cycloalkyl, cycloalkylalkyl, phenyl or benzyl radical, the two latter radicals being optionally substituted with a halogen atom or an alkyl or alkoxy group,
$R_3$ is a hydrogen atom or an alkyl radical,
$R_4$ is a hydrogen atom or an alkyl radical,
said alkyl and alkoxy radicals having 1 to 4 carbon atoms,
with the exception of the compound for which $n = 1$, $R_1 = R_3 = R_4 = H$ and $R_2 = CH_3$,
characterized in that a compound of formula (II)

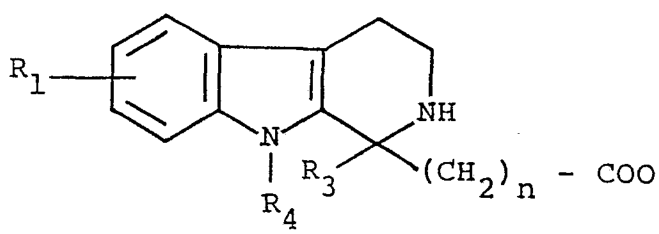

(Et or Me) or

(II)

is reacted with an amine $R_2NH_2$, then the intermediate compound obtained, of formula (III)

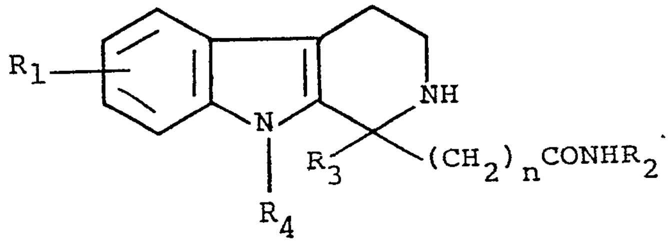

is cyclized by means of formaldehyde in 30% aqueous solution, and, when $R_4 = H$, the corresponding compound (I) may be alkylated to yield a compound (I) wherein $R_4$ is alkyl.